# EUROPEAN PATENT SPECIFICATION

(11) **EP 3 510 797 B1**
(45) Date of publication and mention of the grant of the patent: **10.06.2020**
(21) Application number: 17867753.0
(22) Date of filing: 01.11.2017
(51) Int. Cl.: H04R 25/02, A61N 1/36, A61N 1/05

(54) **ACTUATED ELECTRODE LEAD IN MINIMALLY INVASIVE COCHLEAR IMPLANT SURGERY**
BETÄTIGTE ELEKTRODENLEITUNG FÜR MINIMAL INVASIVE COCHLEACHIRURGIE
FIL D'ÉLECTRODE ACTIONNÉ DANS UNE CHIRURGIE D'IMPLANT COCHLÉAIRE MINIMALEMENT INVASIVE

(30) Priority: 01.11.2016 US 201662415576 P
(43) Date of publication of application: 17.07.2019
(73) Proprietor: Med-El Elektromedizinische Geraete GmbH, 6020 Innsbruck (AT)
(72) Inventor: FRÖLICH, Max, 30449 Hannover (DE); SCHURZIG, Daniel, 30177 Hannover (DE)
(74) Representative: Downing, Michael Philip
(86) International application number: PCT/US2017/059433
(87) International publication number: WO 2018/085319

(56) References cited:
- WO-A1-2014/046662
- US-A- 5 800 500
- US-A1- 2007 005 117
- US-A1- 2009 005 836
- US-A1- 2010 305 676
- US-A1- 2013 023 962
- US-A1- 2016 096 012

## Description

### TECHNICAL FIELD

The present invention relates to medical implants, and more specifically to an implantable electrode arrangement for cochlear implant systems.

### BACKGROUND ART

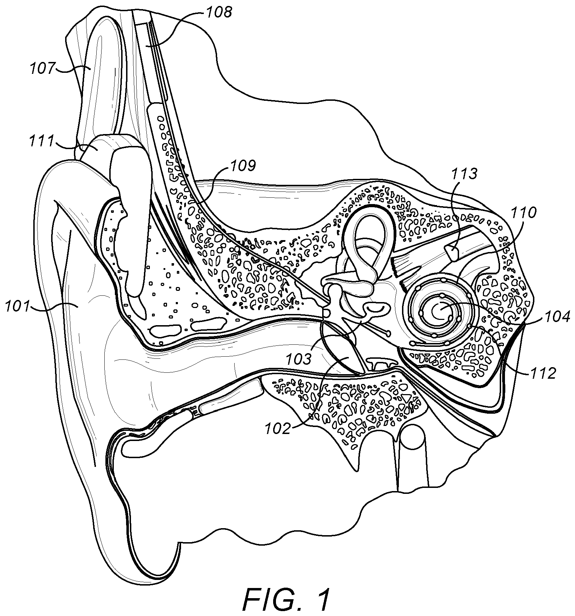

A normal ear transmits sounds as shown in Figure 1 through the outer ear **101** to the tympanic membrane (eardrum) **102,** which moves the bones of the middle ear **103,** which in turn vibrate the oval window and round window openings of the cochlea **104.** The cochlea **104** is a long narrow duct wound spirally about its axis for approximately two and a half turns. The cochlea **104** includes an upper channel known as the scala vestibuli and a lower channel known as the scala tympani, which are connected by the cochlear duct. The scala tympani forms an upright spiraling cone with a center called the modiolar where the spiral ganglion cells of the acoustic nerve **113** reside. In response to received sounds transmitted by the middle ear **103,** the fluid filled cochlea **104** functions as a transducer to generate electric pulses that are transmitted to the cochlear nerve **113,** and ultimately to the brain. Hearing is impaired when there are problems in the ability to transduce external sounds into meaningful action potentials along the neural substrate of the cochlea **104.**

In some cases, hearing impairment can be addressed by an auditory prosthesis system such as a cochlear implant that electrically stimulates auditory nerve tissue with small currents delivered by multiple stimulation contacts distributed along an implant electrode. Figure 1 shows some components of a typical cochlear implant system where an external microphone provides an audio signal input to an external signal processing stage **111** which implements one of various known signal processing schemes. The processed signal is converted by the external signal processing stage **111** into a digital data format, such as a sequence of data frames, for transmission into a receiver processor in an implant housing **108.** Besides extracting the audio information, the receiver processor in the implant housing **108** may perform additional signal processing such as error correction, pulse formation, etc., and produces a stimulation pattern (based on the extracted audio information) that is sent through an electrode lead **109** to an implanted electrode array **110** which penetrates into the cochlea **104** through a surgical opening in the outer surface of the cochlea **104.** Typically, this electrode array **110** includes multiple stimulation contacts **112** on its surface that deliver the stimulation signals to adjacent neural tissue of the cochlea **104** which the brain of the patient interprets as sound. The individual stimulation contacts **112** may be activated sequentially or simultaneously in one or more contact groups.

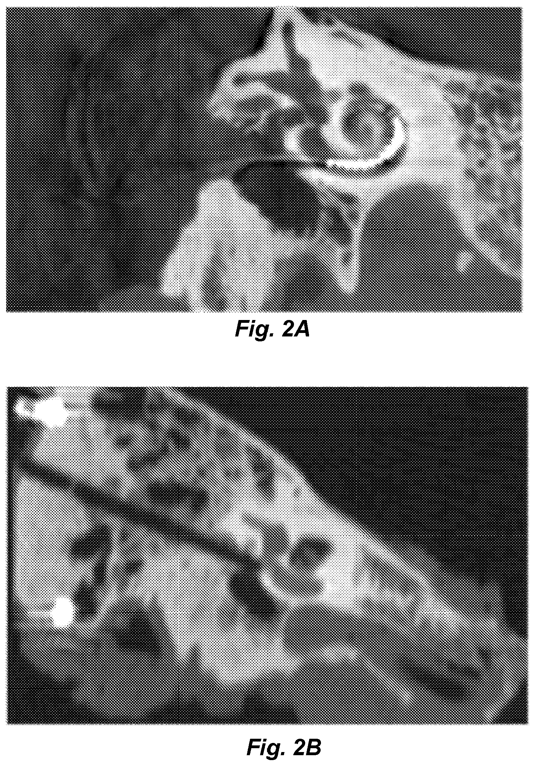

Cochlear implantation is a major surgery that involves full anesthesia and usually takes from 1.5 to 5 hours. A significant portion of that time is required for the labor intensive mastoidectomy in which the surgeon creates an opening in the outer mastoid bone of the skull and an electrode path through that bone and the middle ear to gain access to the cochlea prior to implantation. During this process, the surgeon needs to carefully mill down through the mastoid bone to the cochlea starting right behind the ipsilateral ear, and using anatomical landmarks to find his way. One of these landmarks is the facial nerve which, if damaged or cut, may cause facial paralysis of the patient. Figure 2A shows an x-ray image of a cochlear implant electrode inserted into a patient cochlea via such a conventional mastoidectomy.

Aiming at the reduction of surgery time, patient stress, and risk of accidents such as facial nerve damage, there are research attempts to perform cochlear implantation using image guidance using preoperative CT images for the determination of a single bore path from behind the ear down to the point on the outer surface of the cochlea through which the implant electrode array needs to be inserted. These methods are described in detail, for example, in Labadie et al. "Minimally invasive, image-guided, facial-recess approach to the middle ear: demonstration of the concept of percutaneous cochlear access in vitro." Otology & Neurotology 26.4 (2005): 557-562; which is incorporated herein by reference. Figure 2B shows an x-ray image of a cochlear implant electrode inserted into a patient cochlea via this new minimally invasive technique. While these attempts are known to be very beneficial in terms of the severity of the surgery, the actual insertion of the electrode array into the cochlea becomes significantly more difficult-the geometrical boundary conditions do not allow for visual access of the cochlea opening, and there is little or no space available for surgical insertion mechanisms.

It is known that the length of the electrode lead does not correspond to the exact distance between the final location of the implant housing and the opening into the implanted cochlea. That is because the thickness of the mastoid bone varies between one patient and another. In addition, when using robotic surgery techniques to drill an electrode path to the cochlea, the drilled tunnels are so small that appropriate insertion tools are needed for safe insertion of the electrode array section into the cochlea. These insertion tools usually require some extra length of electrode lead beyond the minimum possible distance between the cochlea and the implant housing to provide appropriate gripping and handling options.

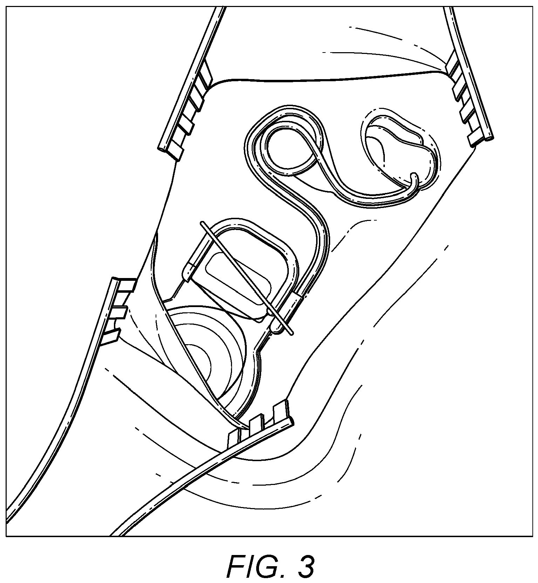

Figure 3 shows the usual surgical technique for storing excess electrode lead in the mastoidectomy opening. The excess lead is looped into an 8- or O-shape in the cavity underneath the mastoid cortical overhang. There appears to be no existing discussion of how to store excess electrode lead length when using the newer minimally invasive bore path technique.

US2016/096012 describes an implantable electrode arrangement for a cochlear implant system. The arrangement includes a flexible intracochlear electrode array having an outer surface with electrode contacts for applying cochlear stimulation signals to target neural tissue within the implanted cochlea. A flexible extracochlear electrode lead is coupled at a lead proximal end to an implanted signal processor that provides the cochlear stimulation signals, and connected at a lead distal end to a proximal end of the electrode array. A lead holder is connected to the lead distal end and has an initial shape. The lead holder is malleable and adapted to be intra-surgically deformed into and retain a new desired shape so as to secure the lead distal end at the electrode opening into the implanted cochlea and decouple post-surgical mechanical strain at the lead distal end from the electrode array.

US5800500 describes a multi-electrode cochlear implant, in which approximately twenty or more insulated metal wires are wound around a flexible tube. These wires are held in place with a further layer of dielectric insulating material. The insulation is selectively removed with a laser beam to form electrodes. Two or more layers or valences of wires can be used, with the inner layer of wires terminating distal to the outer layers to provide a stepwise approximation of the tapering of the scala tympani. A shape memory material core may be introduced into the tube, so that the implant will retain an effective shape after implantation. In a preferred embodiment, electrical conductors are connected to the shape memory material to permit the select warming of the shape memory material by the passing of an electric current through it. In an alternative preferred embodiment, the shape memory material is warmed by adjacent heating elements.

US2010/305676 describes a lead for a cochlear implant, modified so as to provide increased robustness to the lead. In one aspect, the lead has a helix region having an increased length such that it extends into a mastoid cavity of a patient when in situ. In another aspect, the lead has an increased lead angle between the helix region and the transition region.

### SUMMARY

The invention is defined in the appended claims.

Embodiments of the present invention are directed to an implantable electrode arrangement for a cochlear implant system that is suitable for storage of excess electrode lead in a minimally invasive implantation surgery. An implantable electrode arrangement for a cochlear implant system is described. An electrode lead carries one or more cochlear stimulation signals and fits through a mastoid tunnel that extends along a longitudinal tunnel axis from a lateral outer surface of patient mastoid bone through the patient mastoid bone into the middle ear. An electrode array is connected to a distal end of the electrode lead and is inserted into a patient cochlea so that stimulation contacts on its outer surface can apply the cochlear stimulation signals to target neural tissue. A lead structure control element is located within the electrode lead to control a lead shape of the electrode lead between an insertion state wherein the electrode lead within the mastoid tunnel has a longitudinal lead axis lying entirely along the tunnel axis, and a post-insertion state wherein a facial recess portion of the electrode lead fitting within a limited diameter portion of the mastoid tunnel associated with a facial recess portion of the mastoid bone has a longitudinal lead axis along the tunnel axis, and a storage portion of the electrode lead fitting within an enlarged diameter portion of the mastoid tunnel lateral to the facial recess portion has a longitudinal lead axis coiled in a helical shape radially around the tunnel axis.

In further specific embodiments, the lead structure control element may include one or more shape memory alloy elements to control lead shape, and/or the lead structure control element may be configured to control lead shape as a function of temperature. For example, the lead structure control element may include one or more lead heating elements configured for heating the electrode lead.

Specific embodiments of the invention may further include a lead stopper connected to the electrode lead and configured to securely fit into the mastoid tunnel from the middle ear so as to resist rotation of electrode array when the lead structure control element controls the lead shape into the post-insertion state. For example, the lead stopper may be structurally integrated into the electrode lead, or it may be a structurally separate element securely attached to the electrode lead.

The electrode arrangement of the present invention can be surgically implanted in a patient cochlea. An outer mastoid tunnel is prepared that extends along a longitudinal tunnel axis from a lateral outer surface of a patient mastoid bone through the mastoid bone towards a facial recess region of the patient mastoid bone. An inner mastoid tunnel is prepared that extends further along the tunnel axis through the facial recess region of the mastoid bone into the middle ear. The inner mastoid tunnel diameter is less than the outer tunnel diameter. A cochlear opening also is prepared in an outer surface of a patient cochlea at a point along the tunnel axis. An implant electrode arrangement is provided that includes an electrode lead configured for carrying one or more cochlear stimulation signals and having a distal end connected to an electrode array with an outer surface having a plurality of stimulation contacts configured for applying the cochlear stimulation signals to target neural tissue within the patient cochlea. The electrode lead further comprises a lead structure control element configured to control lead shape. The electrode array is fitted through the outer mastoid tunnel, the inner mastoid tunnel, the middle ear, and the cochlear opening to implant the electrode array into the patient cochlea. The lead structure control element operates to maintain the electrode lead enclosed within the outer mastoid tunnel and the inner mastoid tunnel in an insertion shape lying entirely along a longitudinal lead axis extending along the tunnel axis. Then the lead structure control element is operated to modify lead shape of the electrode lead into a post-insertion shape wherein a facial recess portion of the electrode lead fitting within the inner mastoid tunnel has a longitudinal lead axis along the tunnel axis, and a storage portion of the electrode lead fitting within the outer mastoid tunnel has a longitudinal lead axis coiled in a helical shape radially around the tunnel axis.

Embodiments of the present invention also include a cochlear implant system having an electrode arrangement according to any of the foregoing.

### BRIEF DESCRIPTION OF THE DRAWINGS

Figure 1 shows various anatomical structures in a human ear and some components of a typical cochlear implant system.
Figures 2A and 2B show examples of x-ray images for implanting a cochlear implant electrode using a conventional mastoidectomy and a minimally invasive mastoid tunnel respectively.
Figure 3 shows conventional storage of excess electrode lead in a mastoidectomy opening.
Figure 4 shows anatomical details of a mastoid tunnel suitable for excess electrode storage according to an embodiment of the present invention.
Figure 5 shows various logical steps in a method of surgically inserting a cochlear implant electrode array according to an embodiment of the present invention.
Figures 6A-6B show structural details of the surgical insertion process for a cochlear implant electrode according to an embodiment of the present invention.

### DETAILED DESCRIPTION

Embodiments of the present invention are directed to a cochlear implant electrode lead suitable for implementing a new storage technique for excess electrode lead consistent with a minimally invasive implantation approach without needing to create an additional storage cavity.

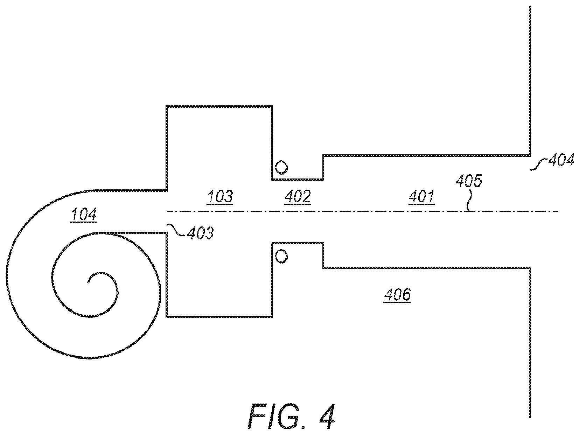

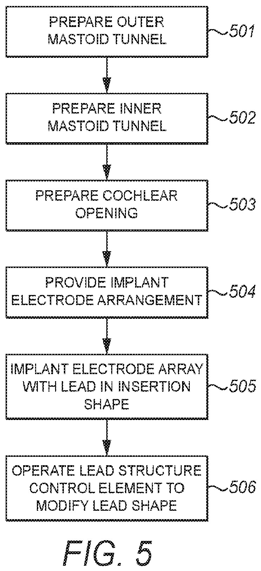

Figure 4 shows anatomical details of a mastoid tunnel **400** suitable for excess electrode storage and Figure 5 shows various logical steps in a method of surgically inserting a cochlear implant electrode array according to an embodiment of the present invention. The mastoid tunnel **400** extends along a longitudinal tunnel axis **405** from a lateral outer surface of patient mastoid bone **404** through the patient mastoid bone **406** into the middle ear **103.** Initially an enlarged diameter outer mastoid tunnel **401** (e.g., about 3.0 mm in diameter) is prepared, step **501,** that is drilled during implantation surgery through the mastoid bone **406** to near the critical facial recess structures of the facial nerve and the chorda tympani, terminating the tunnel before these structures are traumatized or harmed (e.g., about 24.0 mm long). After checking the drilled path, a narrower inner mastoid tunnel **402** (e.g., about 1.8 mm in diameter) then is drilled through the facial recess region of the mastoid bone **406** into the middle ear **103,** step **502.** A cochlear opening **403** is then created in the outer surface of the patient cochlea **104** at a point on the longitudinal tunnel axis **405,** step **503.**

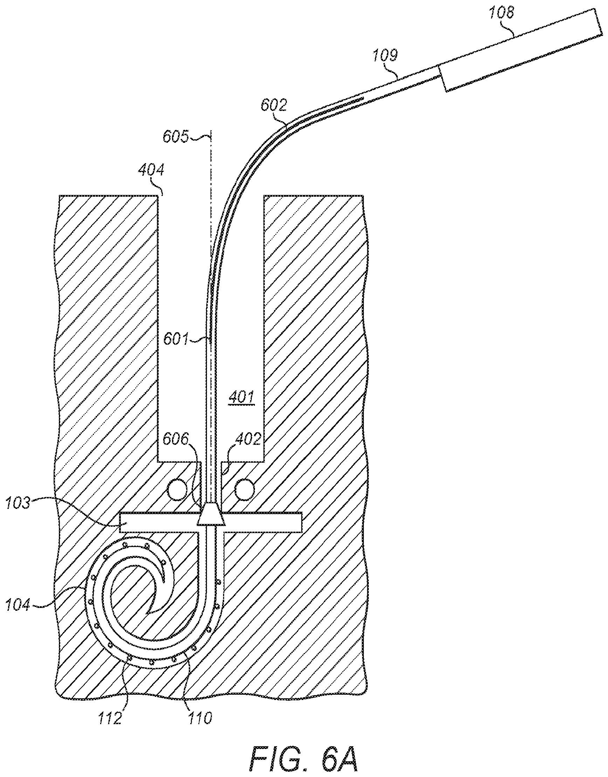

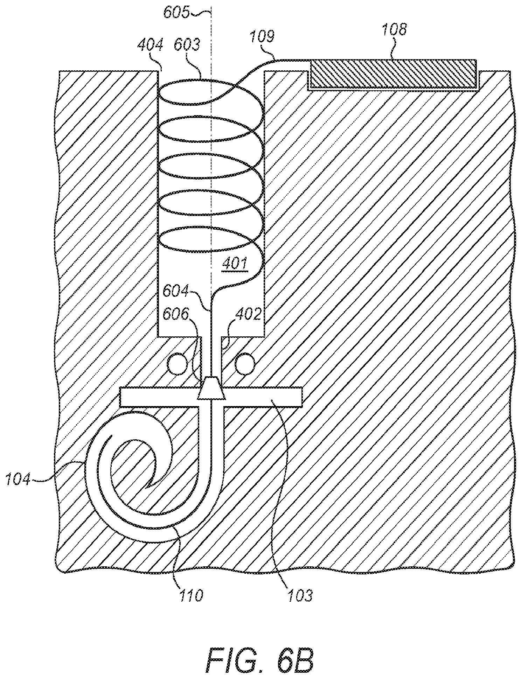

Figures 6A-6B show structural details of the surgical insertion process for a cochlear implant electrode according to an embodiment of the present invention. An implant electrode is provided, step **504,** that includes an electrode lead **109** for carrying one or more cochlear stimulation signals. An electrode array **110** is connected to a distal end of the electrode lead **109** and includes stimulation contacts **112** on its outer surface configure to apply the cochlear stimulation signals to target neural tissue within the implanted cochlea **104.** A lead structure control element **602** also is located within the electrode lead **109** to control the lead shape of the electrode lead **109.** The electrode lead **109** and electrode array **110** are held in an insertion tool (not shown) with the lead control element **602** is operated to maintain an insertion lead shape as shown in Figure 6A wherein the electrode lead **109** within the outer mastoid tunnel **401** and the inner mastoid tunnel **402** has a longitudinal lead axis lying entirely along the tunnel axis **605.** Once the electrode array **110** is fully implanted in the cochlea **104,** step **505,** the insertion tool is removed the implant housing **108** can be secured in its final position. The lead control element **602** is then operated to modify the lead shape into a post-insertion state, step **506,** in which a facial recess portion **604** of the electrode lead **109** within the limited diameter portion of the inner mastoid tunnel **402** continues to have its longitudinal lead axis along the tunnel axis **605,** while a storage portion **603** of the electrode lead **109** fitting within the enlarged diameter outer mastoid tunnel **401** (lateral to the facial recess portion inner mastoid tunnel **402)** has its longitudinal lead axis coiled in a helical shape radially around the tunnel axis **605.**

The lead control element **602** specifically may be composed of one or more shape memory alloy (SMA) elements; for example, materials such as nickel titanium alloys. The SMA elements of the lead control element **602** can be modified out of their "memorized" shape at a first lower temperature. Then the SMA elements can be heated to modify them back into their predefined shape. For example, the heating and the specific transformation temperature of the SMA elements may be based on body temperature. In that case, the SMA elements are passively heated by virtue of their proximity to the surrounding body tissue. Alternatively, the transformation temperature for the SMA elements may lie above body temperature, though preferably only slightly above body temperature. A controlled current then can be applied by one or more dedicated lead heating elements embedded in the electrode lead, where the time and speed of shape transformation can be controlled by the surgeon. The power supply for the control current could be provided via the implant processor or externally. For an external power supply, the extra current supply wire would have to be cut before suturing and closing the implantation wound. Increasing the lead temperature above body temperature during the surgery is acceptable because the heating location is sufficiently far away from the delicate inner ear tissues, which are known to be very sensitive to damage from elevated temperatures. And also the structures that are heated are embedded in the silicone material of the electrode lead, which acts as a heat insulator with a relatively low thermal expansion coefficient.

For the post-insertion state of the stored portion of the electrode lead, the helix shape will automatically start to coil within a defined coiling radius R_{L} like in an old-fashioned telephone handset (e.g., 1.25 mm ≤ R_{L} ≤ 5 mm), which is slightly smaller than the diameter of the outer mastoid tunnel (2R_{L} < T_{D}). The resulting coiling action reduces the length of the electrode lead so that the excess length self-stores into the drilled path of the outer mastoid tunnel, and the implant body can be placed close to the drilled tunnel with no additional drilling of a grove needed.

To control the coiling length and adapt the position of the implant body, the shape structure control element can be divided into multiple different SMA segments, each of which may be controlled separately and which may allow for different coiling radii. In addition or alternatively, the SMA segments can be manually deformed into any desired form and the actuation and shape modification process can be repeated if needed.

It will be appreciated that during the active coiling of the electrode lead, a torque is created that tends to rotate the more distal structures (i.e. the intracochlear electrode array), and/or the more proximal structures (i.e. the implant housing). Rotation of the distal structures needs to be absolutely avoided since rotation of the electrode array within the cochlea would seriously traumatize the delicate tissue therein. To prevent that, embodiments of the invention may include a lead stopper (**606** in Figs. 6A-6B) that is connected to the electrode lead and configured to securely fit into the mastoid tunnel from the middle ear so as to resist rotation of electrode array when the lead structure control element controls the lead shape into the post-insertion state. For example, the lead stopper **605** may be structurally integrated into the electrode lead, or it may be a structurally separate element securely attached to the electrode lead. Once the lead stopper **605** is secured in the tunnel opening, coiling of the storage portion **603** of the electrode lead **109** within the enlarged diameter outer mastoid tunnel **401** creates a rotational force on the entire more proximal portion of the electrode lead **109** and the implant housing **108.** As long as the coiling rate is controlled, the surgeon and/or an appropriate tool can manage this rotational force until the coiling procedure is completed.

The mechanical load onto the electrode lead caused by manipulation by hand and/or surgical tools will be reduced compared to the manipulation in conventional surgical techniques. This is mainly due to the automatic coiling, and thus no additional manual handling is required. And if the coiling radius of the storage portion **603** of the electrode lead **109** within the enlarged diameter outer mastoid tunnel **401** is larger than a critical bending radius for the wires embedded in the electrode lead, there should be no need to consider or account for any breakage of the lead wires.

Although various embodiments of the invention have been disclosed by way of example, it will be apparent to those skilled in the art that various changes and modifications can be made to those examples without departing from the scope of the present invention .

## Claims

1. An implantable electrode arrangement for a cochlear implant system comprising:
an electrode lead (109) configured for carrying one or more cochlear stimulation signals and configured to fit through a mastoid tunnel (401, 402) extending along a longitudinal tunnel axis (605) from a lateral outer surface of patient mastoid bone (404) through the patient mastoid bone (406) into the middle ear (103);
an electrode array (110) connected to a distal end of the electrode lead (109) and configured for insertion into a patient cochlea (104), wherein the electrode array (110) has an outer surface with a plurality of stimulation contacts (112) configured for applying the cochlear stimulation signals to target neural tissue within the patient cochlea (104);
**characterized by** a lead structure control element (602) within the electrode lead (109) configured to control a lead shape of the electrode lead (109) between two stable states:
i. an insertion state wherein the electrode lead (109) within the mastoid tunnel (401, 402) has a longitudinal lead axis lying entirely along the tunnel axis (605);
and
ii. a post-insertion state wherein:
(1) a facial recess portion (604) of the electrode lead (109) fitting within a limited diameter portion (402) of the mastoid tunnel associated with a facial recess portion of the mastoid bone(406) has a longitudinal lead axis along the tunnel axis (605), and
(2) a storage portion (603) of the electrode lead fitting within an enlarged diameter portion (401) of the mastoid tunnel lateral to the facial recess portion has a longitudinal lead axis coiled in a helical shape radially around the tunnel axis (605).

2. The electrode arrangement according to claim 1, wherein the lead structure control element comprises one or more shape memory alloy elements to control lead shape.

3. The electrode arrangement according to any of the preceding claims, wherein the lead structure control element is configured to control lead shape as a function of temperature.

4. The electrode arrangement according to claim 3, wherein the lead structure control element includes one or more lead heating elements configured for heating the electrode lead.

5. The electrode arrangement according to any of the preceding claims, further comprising:
a lead stopper (606) connected to the electrode lead (109) and configured to securely fit into the mastoid tunnel from the middle ear so as to resist rotation of electrode array (110) when the lead structure control element (602) controls the lead shape into the post-insertion state.

6. The electrode arrangement according to claim 5, wherein the lead stopper (606) is structurally integrated into the electrode lead.

7. The electrode arrangement according to claim 5, wherein the lead stopper (606) is a structurally separate element securely attached to the electrode lead (109).

8. A cochlear implant system having an electrode arrangement according to any of claims 1-7.

## Patentansprüche

1. Implantierbare Elektrodenanordnung für ein Cochleaimplantatsystem, umfassend:
eine Elektrodenleitung (109), ausgelegt zum Führen von ein oder mehreren Cochlea-Stimulationssignalen und dafür ausgelegt, durch eine Mastoidhöhle (401, 402) zu passen, die entlang einer Tunnelachse (605) in Längsrichtung von einer seitlichen äußeren Oberfläche eines Mastoidknochens (404) eines Patienten durch den Mastoidknochen (406) des Patienten in das Mittelohr (103) verläuft;
ein Elektroden-Array (110), verbunden mit einem distalen Ende der Elektrodenleitung (109) und ausgelegt zum Einsetzen in eine Cochlea (104) eines Patienten, wobei das Elektroden-Array (110) eine äußere Oberfläche mit einer Mehrzahl von Stimulationskontakten (112) aufweist, ausgelegt zum Beaufschlagen von neuralem Zielgewebe in der Cochlea (104) des Patienten mit den Cochlea-Stimulationssignalen;
**gekennzeichnet durch** ein Leitungsstruktursteuerelement (602) in der Elektrodenleitung (109), ausgelegt zum Steuern einer Leitungsform der Elektrodenleitung (109) zwischen zwei stabilen Zuständen:
i. einem Einsetzzustand, wobei die Elektrodenleitung (109) in der Mastoidhöhle (401, 402) eine Leitungsachse in Längsrichtung aufweist, die vollständig entlang der Tunnelachse (605) liegt; und
ii. einem Zustand nach dem Einsetzen, wobei
(1) ein Aussparungsabschnitt (604) der Elektrodenleitung (109) im Gesicht, der in einen begrenzten Durchmesserabschnitt (402) der Mastoidhöhle passt, in Verbindung mit einem Aussparungsabschnitt des Mastoidknochens (406) im Gesicht eine Leitungsachse in Längsrichtung entlang der Tunnelachse (605) aufweist, und
(2) ein Einlagerungsabschnitt (603) der Elektrodenleitung, der in einen vergrößerten Durchmesserabschnitt (401) der Mastoidhöhle seitlich zu dem Aussparungsabschnitt im Gesicht passt, eine Leitungsachse in Längsrichtung aufweist, die spiralförmig radial um die Tunnelachse (605) gewunden ist.

2. Elektrodenanordnung nach Anspruch 1, wobei das Leitungsstruktursteuerelement ein oder mehrere formspeichernde Legierungselemente zum Steuern der Leitungsform umfasst.

3. Elektrodenanordnung nach einem der vorstehenden Ansprüche, wobei das Leitungsstruktursteuerelement ausgelegt ist zum Steuern der Leitungsform als Funktion der Temperatur.

4. Elektrodenanordnung nach Anspruch 3, wobei das Leitungsstruktursteuerelement ein oder mehrere Leitungsheizelemente aufweist, ausgelegt zum Erwärmen der Elektrodenleitung.

5. Elektrodenanordnung nach einem der vorstehenden Ansprüche, ferner umfassend:
einen Leitungsverschluss (606), verbunden mit der Elektrodenleitung (109) und dafür ausgelegt, sicher in die Mastoidhöhle vom Mittelohr zu passen, um der Rotation des Elektroden-Arrays (110) zu widerstehen, wenn das Leitungsstruktursteuerelement (602) die Leitungsform in den Zustand nach dem Einsetzen steuert.

6. Elektrodenanordnung nach Anspruch 5, wobei der Leitungsverschluss (606) ein in die Elektrodenleitung strukturell integriertes Element ist.

7. Elektrodenanordnung nach Anspruch 5, wobei der Leitungsverschluss (606) ein an die Elektrodenleitung (109) sicher angeschlossenes, strukturell separates Element ist.

8. Cochleaimplantatsystem, aufweisend eine Elektrodenanordnung nach einem der Ansprüche 1-7.

## Revendications

1. Agencement d'électrodes implantables pour un système d'implant cochléaire comprenant :
un fil d'électrode (109) configuré pour transporter un ou plusieurs signaux de stimulation cochléaire et configuré pour s'installer à travers un tunnel mastoïde (401, 402) s'étendant le long d'un axe longitudinal de tunnel (605) depuis une surface externe latérale d'apophyse mastoïde de patient (404) à travers l'apophyse mastoïde de patient (406) jusqu'à l'intérieur de l'oreille moyenne (103) ;
un réseau d'électrodes (110) relié à une extrémité distale du fil d'électrode (109) et configuré pour être inséré dans une cochlée de patient (104), le réseau d'électrodes (110) ayant une surface externe avec une pluralité de contacts de stimulation (112) configurés pour appliquer les signaux de stimulation cochléaire à un tissu neuronal cible à l'intérieur de la cochlée de patient (104) ;
**caractérisé par** un élément de contrôle de structure de fil (602) à l'intérieur du fil d'électrode (109) configuré pour contrôler une forme de fil du fil d'électrode (109) entre deux états stables :
i. un état d'insertion dans lequel le fil d'électrode (109) à l'intérieur du tunnel mastoïde (401, 402) a un axe longitudinal de fil entièrement le long de l'axe de tunnel (605) ; et
ii. un état post-insertion dans lequel :
(1) une partie d'encoche faciale (604) du fil d'électrode (109) s'installant à l'intérieur d'une partie de diamètre limité (402) du tunnel mastoïde associée à une partie d'encoche faciale de l'apophyse mastoïde (406) a un axe longitudinal de fil le long de l'axe de tunnel (605), et
(2) une partie de stockage (603) du fil d'électrode s'installant à l'intérieur d'une partie de diamètre agrandi (401) du tunnel mastoïde latérale de la partie d'encoche faciale a un axe longitudinal de fil enroulé radialement en une forme hélicoïdale autour de l'axe de tunnel (605).

2. Agencement d'électrodes selon la revendication 1, dans lequel l'élément de contrôle de structure de fil comprend un ou plusieurs éléments en alliage à mémoire de forme pour contrôler la forme de fil.

3. Agencement d'électrodes selon l'une quelconque des revendications précédentes, dans lequel l'élément de contrôle de structure de fil est configuré pour contrôler la forme de fil en fonction de la température.

4. Agencement d'électrodes selon la revendication 3, dans lequel l'élément de contrôle de structure de fil comporte un ou plusieurs éléments de chauffage de fil configurés pour chauffer le fil d'électrode.

5. Agencement d'électrodes selon l'une quelconque des revendications précédentes, comprenant en outre :
une butée de fil (606) reliée au fil d'électrode (109) et configurée pour s'installer de façon stable à l'intérieur du tunnel mastoïde depuis l'oreille moyenne de manière à résister à une rotation du réseau d'électrodes (110) quand l'élément de contrôle de structure de fil (602) contrôle la forme de fil jusqu'à l'état post-insertion.

6. Agencement d'électrodes selon la revendication 5, dans lequel la butée de fil (606) est structuralement intégrée dans le fil d'électrode.

7. Agencement d'électrodes selon la revendication 5, dans lequel la butée de fil (606) est un élément structuralement séparé solidement attaché au fil d'électrode (109).

8. Système d'implant cochléaire ayant un agencement d'électrodes selon l'une quelconque des revendications 1 à 7.
